(19) Europäisches Patentamt
European Patent Office
Office européen des brevets

(11) **EP 1 527 683 A1**

(12) **EUROPEAN PATENT APPLICATION**

(43) Date of publication:
**04.05.2005 Bulletin 2005/18**

(51) Int Cl.[7]: **A01N 43/40**
// (A01N43/40, 57:12, 47:04)

(21) Application number: **03356170.5**

(22) Date of filing: **31.10.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Bayer CropScience S.A.**
**69009 Lyon (FR)**

(72) Inventors:
- **Wegmann, Thomas**
  **40764 Lanfenfeld (DE)**
- **Mercer, Richard**
  **69130 Ecully (FR)**
- **Latorse,Marie-Pascale**
  **69490 Saint Romain de Popey (FR)**

(74) Representative: **Nowak, Alexander**
**Bayer CropScience S.A.**
**Département Brevets et Licences**
**14-20, rue Pierre Baizet**
**B.P. 9163**
**69263 Lyon Cedex 09 (FR)**

(54) **Fungicidal composition comprising a pyridylmethylbenzamide derivative and a sulfamide derivative**

(57) A composition comprising at least a pyridylmethylbenzamide derivative of general formula (I) and N-dichlorofluoromethylthio-N',N'-dimethyl-N-p-tolylsulfamide; in an (a) / (b) weight ratio of from 0.01 to 10.

A composition further comprising an additional fungicidal compound.

A method for preventively or curatively combating the phytopathogenic fungi of crops by using this composition.

EP 1 527 683 A1

## Description

The present invention relates to novel fungicidal compositions comprising a pyridylmethylbenzamide derivative and a sulfamide derivative. The present invention also relates to a method of combating phytopathogenic fungi by applying at a locus infested or liable to be infested such a composition.

European patent application EP-A-1056723 generically discloses the possibility of combining pyridylmethybenzamide derivatives with known fungicidal products to develop a fungicidal activity.

International patent application WO 02/069713 discloses fungicidal mixtures comprising a pyridylmethylbenzamide derivative and phosphorous acid or one of its derivatives.

Some of the above mentioned mixtures have shown a synergistic effect. Nevertheless, it is always of high-interest in agriculture to use novel pesticidal mixtures showing a synergistic effect in order to avoid or to control the development of resistant strains to the active ingredients or to the mixtures of known active ingredients used by the farmer while minimising the doses of chemical products spread in the environment and reducing the cost of the treatment.

We have now found some novel fungicidal compositions which possess the above mentioned characteristics.

Accordingly, the present invention relates to a composition comprising :

a) a pyridylmethylbenzamide derivative of general formula (I)

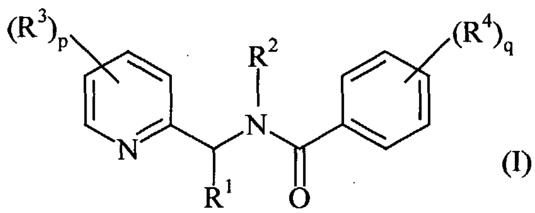

(I)

in which:

- $R^1$ may be a hydrogen atom, an optionally substituted alkyl group or an optionally substituted acyl group;
- $R^2$ may be a hydrogen atom or an optionally substituted alkyl group;
- $R^3$ and $R^4$ may be chosen independently from each other as being a halogen atom, a hydroxyl group, a cyano group, a nitro group, $-SF_5$, a trialkylsilyl group, an optionally substituted amino group, an acyl group, or a group E, OE or SE, in which E may be an alkyl, alkenyl, alkynyl, cycloalkyl, cycloalkenyl, aryl or a heterocyclyl group each of which may optionally be substituted;
- p represents 0, 1, 2, 3 or 4;
- q represents 0, 1, 2, 3 or 4;

and its agriculturally acceptable optical and/or geometric isomers, tautomers and addition salts with an acid or a base;
and
b) N-dichlorofluoromethylthio-N',N'-dimethyl-N-p-tolylsulfamide;

in a (a) / (b) weight ratio of from 0.01 to 10.

N-dichlorofluoromethylthio-N',N'-dimethyl-N-p-tolylsulfamide is a sulfamide derivative fungicide also known as tolylfluanid.

In the context of the present invention :

- the term halogen means bromine, chlorine, iodine or fluorine;
- the term alkyl means a linear or branched saturated hydrocarbon group containing from 1 to 6 carbon atoms;
- the term alkenyl means a linear or branched hydrocarbon group containing from 2 to 6 carbon atoms and an unsaturation in the form of double bond;
- the term alkynyl means a linear or branched hydrocarbon group containing from 2 to 6 carbon atoms and an unsaturation in the form of a triple bond;
- the term alkoxy means a linear or branched alkyloxy group containing from to 1 to 6 carbon atoms;
- the term acyl means a formyl group or linear or branched alkoxycarbonyl group containing from 2 to 6 carbon atoms;
- the term cycloalkyl means a saturated cyclic hydrocarbon group containing from 3 to 8 carbon atoms;

- the term aryl means a phenyl or naphthyl group;
- the term heterocyclyl means saturated, partially saturated, unsaturated or aromatic cyclic group containing from 3 to 8 atoms, which may be a carbon atom, a nitrogen atom, a sulphur atom or an oxygen atom. Examples of such hetercoyclyl may be pyridyl, pyridinyl, quinolyl, furyl, thienyl, pyrrolyl, oxazolinyl;
- the term "optionally substituted " means that the group thus termed may be substituted with one or more groups which may be halogen, alkyl, alkoxy, hydroxyl, nitro, amin, cyano or acyl.

The composition according to the present invention provides a synergistic effect. This synergistic effect allows a reduction of the chemical substances spread into the environment and a reduction of the cost of the fungal treatment.

In the context of the present invention, the term "synergistic effect" is defined by Colby according to the article entitled "Calculation of the synergistic and antagonistic responses of herbicide combinations" Weeds, (1967), 15, pages 20-22.

The latter article mentions the formula:

$$E = x + y - \frac{x * y}{100}$$

in which E represents the expected percentage of inhibition of the disease for the combination of the two fungicides at defined doses (for example equal to x and y respectively), x is the percentage of inhibition observed for the disease by the compound (I) at a defined dose (equal to x), y is the percentage of inhibition observed for the disease by the compound (II) at a defined dose (equal to y). When the percentage of inhibition observed for the combination is greater than E, there is a synergistic effect.

The composition according to the present invention comprises a pyridylmethylbenzamide derivative of general formula (I).

Preferably, the present invention relates to a composition comprising a pyridylmethylbenzamide derivative of general formula (I) in which the different characteristics may be chosen alone or in combination as being :

- as regards $R^1$ and $R^2$, $R^1$ and $R^2$ may be chosen independently from each other as being a hydrogen atom or an optionally substituted alkyl group. More preferably, R' and $R^2$ may be chosen independently from each other as being a hydrogen atom, a methyl group or an ethyl group. Even more preferably, R' and $R^2$ may be both hydrogen atoms.
- as regards $R^3$ and $R^4$, $R^3$ and $R^4$ may be chosen independently from each other as being a halogen atom, a hydroxyl group, a nitro group, an optionally substituted amino group, an acyl group, or a group E, OE or SE, in which E may be a alkyl, a cycloalkyl, a phenyl or a heterocyclyl group, each of which may optionally be subtituted. More preferably, $R^3$ and $R^4$ may be chosen independently from each other as being a halogen atom, a nitro group or a halogenoalkyl group. Even more preferably $R^3$ and $R^4$ may be chosen independently from each other as being a chlorine atom, a nitro group or a trifluoromethyl group.
- as regards p, p may be 1 or 2. More preferably, p may be 2.
- as regards q, q may be 1 or 2. More preferably, q may be 2;

and its agriculturally acceptable possible tautomers and addition salts with an acid or a base.

More preferably, the pyridylmethylbenzamide derivative of general formula (I) present in the composition of the present invention is :

- a compound (Ia) which is 2,6-dichloro-N-{[3-chloro-5-(trifluoromethyl)-2-pyridinyl]methyl}benzamide; or
- a compound (Ib) which is N-{[3-chloro-5-(trifluoromethyl)-2-pyridinyl]methyl}-2-fluoro-6-nitrobenzamide; or
- a compound (Ic) which is N-{[3-chloro-5-(trifluoromethyl)-2-pyridinyl]methyl}-2-methyl-6-nitrobenzamide;

and its agriculturally acceptable possible tautomers and addition salts with an acid or a base.

The composition according to the present invention comprises at least a pyridylmethylbenzamide derivative of general formula (I) (a) and tolylfluanid (b) in an (a) / (b) weight ratio of from 0.01 to 10; preferably of from 0.05 to 0.5; even more preferably, of from 0.1 to 0.2.

The composition of the present invention may further comprise a third fungicide active ingredient (c).

The third fungicidal active ingredient may preferably be selected from phosphorous acid derivative, phosphorous acid itself, or alkali metal, alkaline-earth metal or metallic salts thereof. More preferably, the additional fungicidal compound may be chosen as being ethyl hydrogen phosphonate.

Ethyl hydrogen phosphonate is a fungicidal compound also known as Fosetyl-Al.

Where the third active ingredient (c) as defined above is present in the composition, this compound may be

present in an amount of (a) : (b) : (c) weight ratio of from 0.01 : 1 : 0.1 to 10 : 1 : 10; the ratios of compound (a) and compound (c) varying independently from each other. Preferably, the (a) : (b) : (c) weight ratio may be of from 0.05 : 1 : 0.5 to 0.5 : 1 : 5; more preferably of from 0.1 : 1 : 1 to 0.2 : 1 : 4.

The composition according to the present invention may further comprise an other additional component such as an agriculturally acceptable support, carrier or filler.

In the present specification, the term "support" denotes a natural or synthetic, organic or inorganic material with which the active material is combined to make it easier to apply, notably to the parts of the plant. This support is thus generally inert and should be agriculturally acceptable. The support may be a solid or a liquid. Examples of suitable supports include clays, natural or synthetic silicates, silica, resins, waxes, solid fertilisers, water, alcohols, in particular butanol, organic solvents, mineral and plant oils and derivatives thereof. Mixtures of such supports may also be used.

The composition may also comprise other additional components. In particular, the composition may further comprise a surfactant. The surfactant can be an emulsifier, a dispersing agent or a wetting agent of ionic or non-ionic type or a mixture of such surfactants. Mention may be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyols, and derivatives of the above compounds containing sulphate, sulphonate and phosphate functions. The presence of at least one surfactant is generally essential when the active material and/or the inert support are water-insoluble and when the vector agent for the application is water. Preferably, surfactant content may be comprised between 5% and 40% by weight of the composition.

Additional components may also be included, e.g. protective colloids, adhesives, thickeners, thixotropic agents, penetration agents, stabilisers, sequestering agents. More generally, the active materials can be combined with any solid or liquid additive, which complies with the usual formulation techniques.

In general, the composition according to the invention may contain from 0.05 to 99% (by weight) of active material, preferably 10 to 70% by weight.

Compositions according to the present invention can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure), gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ulv) liquid, ultra low volume (ulv) suspension, water dispersible granules or tablets, water dispersible powder for slurry treatment, water soluble granules or tablets, water soluble powder for seed treatment and wettable powder.

These compositions include not only compositions which are ready to be applied to the plant or seed to be treated by means of a suitable device, such as a spraying or dusting device, but also concentrated commercial compositions which must be diluted before they are applied to the crop.

The fungicidal compositions of the present invention can be used to curatively or preventively control the phytopathogenic fungi of crops. Thus, according to a further aspect of the present invention, there is provided a method for preventively or curatively controlling phytopathogenic fungi of crops characterised in that a fungicidal composition as hereinbefore defined is applied to the seed, the plant and/or to the fruit of the plant or to the soil in which the plant is growing or in which it is desired to grow.

The composition as used against phytopathogenic fungi of crops comprises an effective and non-phytotoxic amount of an active material of general formula (I).

The expression "effective and non-phytotoxic amount" means an amount of composition according to the invention which is sufficient to control or destroy the fungi present or liable to appear on the crops, and which does not entail any appreciable symptom of phytotoxicity for the said crops. Such an amount can vary within a wide range depending on the fungus to be combated, the type of crop, the climatic conditions and the compounds included in the fungicidal composition according to the invention.

This amount can be determined by systematic field trials, which are within the capabilities of a person skilled in the art.

The method of treatment according to the present invention is useful to treat propagation material such as tubers or rhizomes, but also seeds, seedlings or seedlings pricking out and plants or plants pricking out. This method of treatment can also be useful to treat roots. The method of treatment according to the present invention can also be useful to treat the overground parts of the plant such as trunks, stems or stalks, leaves, flowers and fruits of the concerned plant.

Among the plants that can be protected by the method according to the invention, mention may be made of cotton; flax; vine; fruit crops such as *Rosaceae sp*. (for instance pip fruits such as apples and pears, but also stone

fruits such as apricots, almonds and peaches), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for instance banana trees and plantins), *Rubiaceae sp., Theaceae sp., Sterculiceae sp., Rutaceae sp.* (for instance lemons, oranges and grapefruits); leguminous crops such as *Solanaceae sp.* (for instance tomatoes), *Liliaceae sp., Asteraceae sp.* (for instance lettuces), *Umbelliferae sp., Cruciferae sp., Chenopodiaceae sp., Cucurbitaceae sp., Papilionaceae sp.* (for instance peas), *Rosaceae sp.* (for instance strawberries); big crops such as *Graminae sp.* (for instance maize, cereals such as wheat, rice, barley and triticale), *Asteraceae sp.* (for instance sunflower), *Cruciferae sp.* (for instance colza), *Papilionaceae sp.* (for instance soja), *Solanaceae sp.* (for instance potatoes), *Chenopodiaceae sp.* (for instance beetroots); horticultural and forest crops; as well as genetically modified homologues of these crops.

Among the plants and the possible diseases of these plants protected by the method according to the present invention, mention may be made of :

- wheat, as regards controlling the following seed diseases: fusaria (*Microdochium nivale* and *Fusarium roseum*), stinking smut (*Tilletia caries, Tilletia controversa* or *Tilletia indica*), septoria disease (*Septoria nodorum*) and loose smut;
- wheat, as regards controlling the following diseases of the aerial parts of the plant: cereal eyespot (*Tapesia yallundae, Tapesia acuiformis*), take-all (*Gaeumannomyces graminis*), foot blight (*F. culmorum, F. graminearum*), black speck (*Rhizoctonia cerealis*), powdery mildew (*Erysiphe graminis forma specie tritici),* rusts (*Puccinia striiformis* and *Puccinia recondita*) and septoria diseases (*Septoria tritici* and *Septoria nodorum*);
- wheat and barley, as regards controlling bacterial and viral diseases, for example barley yellow mosaic;
- barley, as regards controlling the following seed diseases: net blotch (*Pyrenophora graminea, Pyrenophora teres* and *Cochliobolus sativus*), loose smut (*Ustilago nuda*) and fusaria (*Microdochium nivale* and *Fusarium roseum*);
- barley, as regards controlling the following diseases of the aerial parts of the plant: cereal eyespot (*Tapesia yallundae*), net blotch (*Pyrenophora teres* and *Cochliobolus sativus*), powdery mildew (*Erysiphe graminis forma specie hordei),* dwarf leaf rust (*Puccinia hordei*) and leaf blotch (*Rhynchosporium secalis*);
- potato, as regards controlling tuber diseases (in particular *Helminthosporium solani, Phoma tuberosa, Rhizoctonia solani, Fusarium solani*), mildew (*Phytopthora infestans*) and certain viruses (virus Y);
- potato, as regards controlling the following foliage diseases: early blight (*Alternaria solani*), mildew (*Phytophthora infestans*);
- cotton, as regards controlling the following diseases of young plants grown from seeds: damping-off and collar rot (*Rhizoctonia solani, Fusarium oxysporum*) and black root rot (*Thielaviopsis basicola*);
- protein yielding crops, for example peas, as regards controlling the following seed diseases: anthracnose (*Ascochyta pisi, Mycosphaerella pinodes*), fusaria (*Fusarium oxysporum*), grey mould (*Botrytis cinerea)* and mildew (*Peronospora pisi*);
- oil-bearing crops, for example rape, as regards controlling the following seed diseases: *Phoma lingam, Alternaria brassicae* and *Sclerotinia sclerotiorum;*
- corn, as regards controlling seed diseases: (*Rhizopus* sp., *Penicillium* sp., *Trichoderma* sp., *Aspergillus* sp., and *Gibberella fujikuroi*);
- flax, as regards controlling the seed disease: *Alternaria linicola;*
- forest trees, as regards controlling damping-off (*Fusarium oxysporum, Rhizoctonia solani*);
- rice, as regards controlling the following diseases of the aerial parts: blast disease *(Magnaporthe grisea*), bordered sheath spot (*Rhizoctonia solani);*
- leguminous crops, as regards controlling the following diseases of seeds or of young plants grown from seeds: damping-off and collar rot (*Fusarium oxysporum, Fusarium roseum, Rhizoctonia solani, Pythium sp.*);
- leguminous crops, as regards controlling the following diseases of the aerial parts: grey mould (Botrytis sp.), powdery mildews (in particular *Erysiphe cichoracearum, Sphaerotheca fuliginea* and *Leveillula taurica*), fusaria (*Fusarium oxysporum*, *Fusarium roseum*), leaf spot (*Cladosporium sp.*), alternaria leaf spot (*Alternaria sp.*), anthracnose (*Colletotrichum sp.*), septoria leaf spot (*Septoria sp.*), black speck (*Rhizoctonia solani*), mildews (for example *Bremia lactucae, Peronospora sp., Pseudoperonospora sp., Phytophthora sp.*);
- fruit trees, as regards diseases of the aerial parts: monilia disease (*Monilia fructigenae, M. laxa*), scab (*Venturia inaequalis*), powdery mildew (*Podosphaera leucotricha*);
- vine, as regards diseases of the foliage: in particular grey mould (*Botrytis cinerea*), powdery mildew (*Uncinula necator*), black rot (*Guignardia biwelli*) and mildew (*Plasmopara viticola*);
- beetroot, as regards the following diseases of the aerial parts: cercospora blight (*Cercospora beticola*), powdery mildew (*Erysiphe beticola*), leaf spot (*Ramularia beticola*).

Preferably, the plant that can be protected by the method according to the present invention is vine.

The fungicidal composition according to the present invention may also be used against fungal diseases liable

to grow on or inside timber. The term "timber" means all types of species of wood, and all types of working of this wood intended for construction, for example solid wood, high-density wood, laminated wood, and plywood. The method for treating timber according to the invention mainly consists in contacting one or more compounds of the present invention, or a composition according to the invention; this includes for example direct application, spraying, dipping, injection or any other suitable means.

The dose of active material usually applied in the treatment according to the present invention is generally and advantageously between 10 and 2000 g/ha, preferably between 50 and 1500 g/ha for applications in foliar treatment. The dose of active substance applied is generally and advantageously between 2 and 200 g per 100 kg of seed, preferably between 3 and 150 g per 100 kg of seed in the case of seed treatment. It is clearly understood that the doses indicated above are given as illustrative examples of the invention. A person skilled in the art will know how to adapt the application doses according to the nature of the crop to be treated.

The fungicidal composition according to the present invention may also be used in the treatment of genetically modified organisms with the compounds according to the invention or the agrochemical compositions according to the invention. Genetically modified plants are plants into whose genome a heterologous gene encoding a protein of interest has been stably integrated. The expression "heterologous gene encoding a protein of interest" essentially means genes which give the transformed plant new agronomic properties, or genes for improving the agronomic quality of the transformed plant.

The compositions according to the present invention may also be used fore the preparation of composition useful to curatively or preventively treat human and animal fungal diseases such as, for example, mycoses, dermatoses, trichophyton diseases and candidiases or diseases caused by *Aspergillus spp.,* for example *Aspergillus fumigatus*.

## Claims

1. A composition comprising :

   a) a pyridylmethylbenzamide derivative of general formula (I)

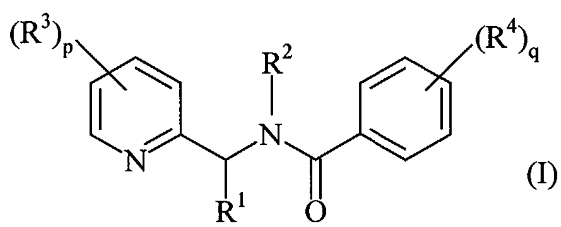

(I)

   in which:

   - $R^1$ may be a hydrogen atom, an optionally substituted alkyl group or an optionally substituted acyl group;
   - $R^2$ may be a hydrogen atom or an optionally substituted alkyl group;
   - $R^3$ and $R^4$ may be chosen independently from each other as being a halogen atom, a hydroxyl group, a cyano group, a nitro group, $-SF_5$, a trialkylsilyl group, an optionally substituted amino group, an acyl group, or a group E, OE or SE, in which E may be an alkyl, alkenyl, alkynyl, cycloalkyl, cycloalkenyl, aryl or a heterocyclyl group each of which may optionally be substituted;
   - p represents 0, 1, 2, 3 or 4;
   - q represents 0, 1, 2, 3 or 4;

   and its agriculturally acceptable optical and/or geometric isomers, tautomers and addition salts with an acid or a base;
   and
   b) N-dichlorofluoromethylthio-N',N'-dimethyl-N-p-tolylsulfamide;

   in an (a) / (b) weight ratio of from 0.01 to 10.

2. A composition according to claim 1, **characterised in that** $R^1$ and $R^2$ are chosen independently from each other as being a hydrogen atom or an optionally substituted alkyl group.

**3.** A composition according to claim 3, **characterised in that** $R^1$ and $R^2$ are both hydrogen atoms.

**4.** A composition according to any one of the claims 1 to 3, **characterised in that** $R^3$ and $R^4$ are chosen independently from each other as being a halogen atom, a hydroxyl group, a nitro group, an optionally substituted amino group, an acyl group, or a group E, OE or SE, in which E may be a alkyl, a cycloalkyl, a phenyl or a heterocyclyl group, each of which may optionally be subtituted.

**5.** A composition according to claim 4, **characterised in that** $R^3$ and $R^4$ are chosen independently from each other as being a halogen atom, a nitro group or a halogenoalkyl group.

**6.** A composition according to claim 5, **characterised in that** the halogen atom is a chlorine atom and the halogenoalkyl group is a trifluoromethyl group.

**7.** A composition according to any one of the claims 1 to 6, **characterised in that** p and q are chosen independently from each other as being is 1 or 2.

**8.** A composition according to claim 7, **characterised in that** p is 2.

**9.** A composition according to claim 7 or 8, **characterised in that** q is 2.

**10.** A composition according to any one of the claims 1 to 9, **characterised in that** the compound of general formula (I) is chosen as being

- a compound (Ia) which is 2,6-dichloro-N-{[3-chloro-5-(trifluoromethyl)-2-pyridinyl]methyl}benzamide; or
- a compound (Ib) which is N-{[3-chloro-5-(trifluoromethyl)-2-pyridinyl]methyl}-2-fluoro-6-nitrobenzamide; or
- a compound (Ic) which is N-{[3-chloro-5-(trifluoromethyl)-2-pyridinyl]methyl}-2-methyl-6-nitrobenzamide.

**11.** A composition according to any one of the claims 1 to 10, **characterised in that** the (a) / (b) weight ratio is from 0.05 to 0.5.

**12.** A composition according to claim 11, **characterised in that** the (a) / (b) weight ratio is from 0.1 to 0.2.

**13.** A composition according to any one of the claims 1 to 12 further comprising a fungicidal compound (c).

**14.** A composition according to claim 13, **characterised in that** the additional fungicidal compound is selected from phosphorous acid derivative, phosphorous acid itself, or alkali metal, alkaline-earth metal or metallic salts thereof.

**15.** A composition according to claim 14, **characterised in that** the additional fungicidal compound is ethyl hydrogen phosphonate.

**16.** A composition according to any one of the claims 13 to 15, **characterised in that** compound (c) is present in an amount of (a) : (b) : (c) weight ratio of from 0.01 : 1 : 0.1 to 10 : 1 : 10; the ratios of compound (a) and compound (c) varying independently from each other.

**17.** A composition according to any one of the claims 1 to 16, **characterised in that** it further comprises an agriculturally acceptable support, carrier, filler and/or surfactant.

**18.** A method for preventively or curatively controlling phytopathogenic fungi of crops, **characterised in that** an effective and non-phytotoxic amount of a composition according to any one of the claims 1 to 17 is applied to the seed, the plant and/or to the fruit of the plant or to the soil in which the plant is growing or in which it is desired to grow.

**19.** A method according to claim 18, **characterised in that** the plant is vine.

European Patent Office

# EUROPEAN SEARCH REPORT

Application Number
EP 03 35 6170

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 03/034824 A (BAYER CROPSCIENCE S A ;MERCER RICHARD (FR); WEGMANN THOMAS (FR)) 1 May 2003 (2003-05-01)<br>* page 5, line 14 - page 13, line 15 *<br>--- | 1-19 | A01N43/40<br>//(A01N43/40, 57:12,47:04) |
| A | WO 99/31981 A (SCHELBERGER KLAUS ;BASF AG (DE); EICKEN KARL (DE); LORENZ GISELA () 1 July 1999 (1999-07-01)<br>* page 1, line 6 - line 40 *<br>* page 3, line 18 - line 28 *<br>--- | 1-19 | |
| D,A | WO 02/069713 A (LATORSE MARIE-PASCALE ;MERCER RICHARD (FR); AVENTIS CROPSCIENCE SA) 12 September 2002 (2002-09-12)<br>* page 2, line 19 - page 8, line 14 *<br>----- | 1-19 | |
| | | | TECHNICAL FIELDS SEARCHED (Int.Cl.7) |
| | | | A01N |

The present search report has been drawn up for all claims

1

| Place of search | Date of completion of the search | Examiner |
|---|---|---|
| THE HAGUE | 13 May 2004 | Lamers, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 35 6170

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2004

| Patent document cited in search report | Publication date | Patent family member(s) | Publication date |
|---|---|---|---|
| WO 03034824 A | 01-05-2003 | FR 2831022 A1 | 25-04-2003 |
| | | WO 03034824 A1 | 01-05-2003 |
| WO 9931981 A | 01-07-1999 | AT 222695 T | 15-09-2002 |
| | | AU 754336 B2 | 14-11-2002 |
| | | AU 2413999 A | 12-07-1999 |
| | | BR 9813681 A | 10-10-2000 |
| | | CA 2313322 A1 | 01-07-1999 |
| | | CN 1282208 T | 31-01-2001 |
| | | DE 59805354 D1 | 02-10-2002 |
| | | DK 1039803 T3 | 14-10-2002 |
| | | EA 3284 B1 | 24-04-2003 |
| | | WO 9931981 A2 | 01-07-1999 |
| | | EP 1201126 A2 | 02-05-2002 |
| | | EP 1039803 A2 | 04-10-2000 |
| | | ES 2183441 T3 | 16-03-2003 |
| | | HU 0100717 A2 | 28-06-2001 |
| | | JP 2001526189 T | 18-12-2001 |
| | | NZ 505625 A | 29-04-2003 |
| | | PL 342400 A1 | 04-06-2001 |
| | | PT 1039803 T | 31-01-2003 |
| | | SI 1039803 T1 | 31-10-2002 |
| | | SK 8132000 A3 | 12-03-2001 |
| | | TW 450787 B | 21-08-2001 |
| | | US 2003036480 A1 | 20-02-2003 |
| | | US 6365608 B1 | 02-04-2002 |
| | | ZA 9811555 A | 19-06-2000 |
| WO 02069713 A | 12-09-2002 | FR 2821720 A1 | 13-09-2002 |
| | | BR 0207999 A | 02-03-2004 |
| | | CA 2439550 A1 | 12-09-2002 |
| | | EP 1372393 A1 | 02-01-2004 |
| | | WO 02069713 A1 | 12-09-2002 |
| | | HU 0303425 A2 | 01-03-2004 |

EPO FORM P0459

For more details about this annex : see Official Journal of the European Patent Office, No. 12/82